Europäisches Patentamt

European Patent Office

Office européen des brevets

(11) Publication number: **0 331 457**
**A2**

# EUROPEAN PATENT APPLICATION

(21) Application number: **89302026.3**

(22) Date of filing: **01.03.89**

(51) Int. Cl.⁴: **A 61 F 2/16**

(30) Priority: **04.03.88 US 164140**

(43) Date of publication of application:
**06.09.89 Bulletin 89/36**

(84) Designated Contracting States:
**AT BE CH DE ES FR GB GR IT LI LU NL SE**

(71) Applicant: **ALCON SURGICAL, INC.,**
**6201 South Freeway**
**Forth Worth Texas 76134 (US)**

(72) Inventor: **Yang, Shih-Liang**
**4620 193rd Place, S.E.**
**Issaquah, Washington 98027 (US)**

(74) Representative: **Harrison, Philippa Dinah et al**
**A. A. THORNTON & CO Northumberland House 303-306**
**High Holborn**
**London WC1V 7LE (GB)**

(54) Method for making lens with colored periphery.

(57) A method of forming a composite lens blank (B) from which a lens (10, 18) adapted for use with the human eye can be formed is disclosed. The method includes providing a first portion (28, 42, 58) formed of a solid, biocompatible plastic that can be sterilized. A liquid biocompatible plastic is then positioned adjacent to the first portion (28, 42, 58) to form a second portion (36, 50, 64). The plastics are selected so that the first and second portions will be bonded through polymerization. The positioning of the portions is arranged so that the material of the outer portion (36, 42, 58) is colored and the material of the inner portion (28, 50, 64) is optically transparent. Upon hardening the material of the second portion (36, 50, 64) forms a polymerized composite (c) with the first portion (28, 42, 58).

FIG.6A  FIG.6B  FIG.6C  FIG.6D

EP 0 331 457 A2

Bundesdruckerei Berlin

## Description

## METHOD FOR MAKING LENS WITH COLORED PERIPHERY

### Technical Field of the Invention

This invention relates to lenses and particularly lenses that are adapted to be implanted in the eye, including intraocular lenses (IOLs) that are used to replace the natural lens of the eye and intracorneal lenses (ICLs) that are adapted to be implanted in the cornea of the eye. More particularly, this invention relates to processes or methods for forming such IOLs or ICLs with colored peripheral portions such as a portion of the periphery of the optic portion of the lens or, in the case of IOLs, colored supporting loops or haptics.

### Description of Background

IOLs of a number of different designs have been developed to replace the natural lens of an eye after it has been removed through known surgical procedures. Although early IOLs were designed to be sutured to or otherwise supported by the iris, most modern IOLs are held in place through support loops or haptics that project from an optical portion of the lens and engage the interior of the eye either on the anterior or posterior side of the iris. Such IOLs have typically been formed by attaching strands of flexible material to an optical portion to form what is known as a multi-piece IOL, or by milling or otherwise cutting an IOL including the supporting loops from a single piece of clear plastic material.

IOLs with colored haptics have been used to aid a physician in observing an maneuvering an IOL during the delicate and intricate surgical implantation procedure. This was relatively easy to accomplish with multi-piece IOLs because colored support loops could readily be attached to the optical portion of the lens. This was more difficult to do with IOLs milled from a single piece of material since the colored portion would have to be permanently attached to the optical portion before the lens is formed.

It has also been suggested to form a colored rim around the optical portion of an IOL in order to reduce glare. Such a feature would also aid in implantation because the optical portion would be more readily visible to a physician.

An early disclosure of an IOL with colored haptics is in a Rayner and Keller catalogue lens style sheet, dated July 31, 1978 (623-6), which illustrates an IOL formed of a single-piece of plastic, with large rigid haptic portions that are colored or opaque.

U.S. patent 4,676,791 to LeMaster et al., teaches various processes for forming a plastic blank with a ring or rim of colored material so that IOLs can be fabricated with colored supporting loops and a colored rim around the IOL optical portion. Lens blanks are formed from an elongated rod in the form of a composite of a transparent inner portion and a colored outer portion which is fabricated by various extrusion methods or by applying dye to the outer surface of a clear rod.

The extrusion process includes applying a layer of colored plastic to a plastic boule by passing the boule through an extrusion dye having a selected diameter to apply the colored layer at a desired thickness. Alternatively, a colored layer is fabricated as a tube and a clear inner portion applied by extruding it into the clear portion of the tube. The resulting article is then heated and drawn to a desired diameter from which the blanks can be cut.

IOLs are formed by attaching support loops to the blanks or by forming the outer colored rim wide enough so that support loops can be formed on a lathe as an integral part of the optical portion. The outer colored portion can be uniformly colored or graded with the color being darker toward the outer edge.

This extrusion process is limited in the number of materials that can be used. Existing extrudable grade PMMA polymers are the only ones that can be used. This means that many materials such as clear Perspex CQ PMMA from ICI and UV-absorbing PMMA, UF-4, from Rohm & Haas which are currently being used in IOL industry as optic materials are not feasible in such a process. Both Perspex CQ and UF-4 PMMA have much higher molecular weight compared with extrudable PMMA. Further, an extrusion process only provides a mechanical bond between the optic portion and rim, the integrity of which is dependent on the consistency and compatibility of the materials and the extrusion parameters, such as extrusion rate and pressure.

### SUMMARY OF THE INVENTION

Briefly, the present invention is directed to various improved methods of forming IOLs or ICLs with a ring or rim of colored material around the optical portion. For IOLs, support loops of colored material can also be formed integral with the optical portion. The improved methods of joining the colored and transparent portions include polymerization of a central clear portion and a peripheral colored portion.

### BRIEF DESCRIPTION OF THE DRAWINGS

A better understanding of the invention can be obtained when the "Detailed Description of Exemplary Embodiments" set forth below is considered in conjunction with the appended drawings, in which:

Figures 1-4 illustrate various types of intraocular lenses that can be formed in accordance with the methods of the present invention;

Figure 5 illustrates lens blanks of different shapes which can be formed in accordance with the invention;

Figure 6 illustrates schematically one polymerization process for forming lens blanks in accordance with the present invention, where colored monomer mixture is poured into a mole and polymerized to the outer surface of a clear rod;

Figure 7 illustrates schematically an alternative process for fabricating lens blanks in accordance with the present invention where a central opening is drilled in a plastic rod and filled with clear plastic material that is polymerized to the surrounding material;

Figure 8 illustrates schematically another alternative embodiment of the invention where openings are formed in a sheet of colored plastic material and a clear plastic monomer mixture poured in the openings and polymerized to surrounding material; and

Figure 9 illustrates schematically another alternative embodiment of the invention where two separate colored outer portions are polymerized to a clear central portion.

## DETAILED DESCRIPTION OF EXEMPLARY EMBODIMENTS

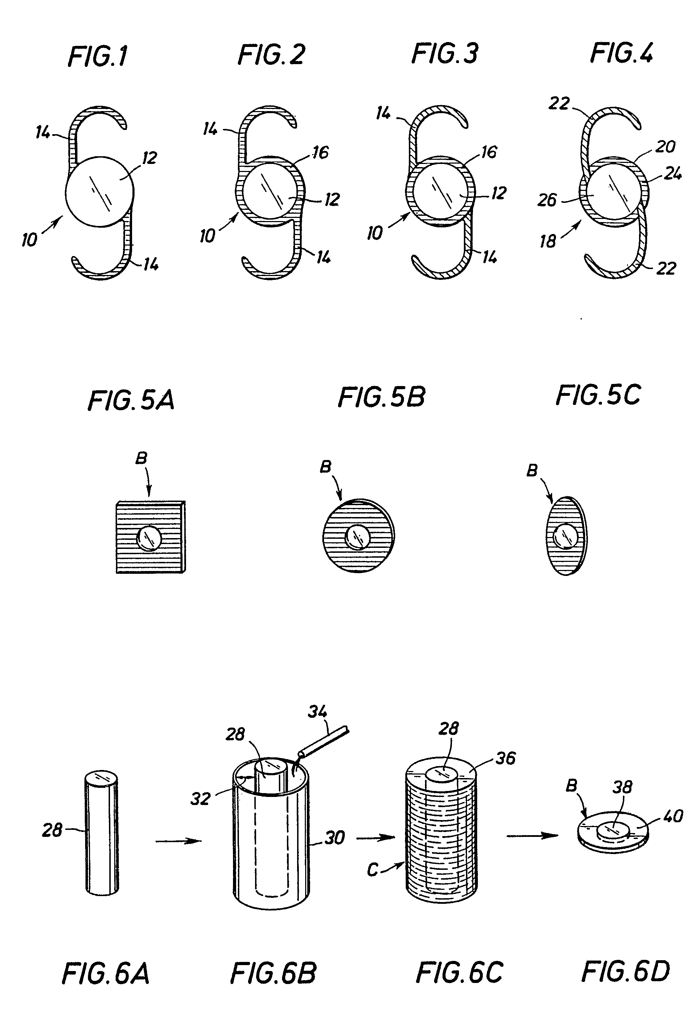

Before describing different methods within the scope of the present invention, reference should be made to Figs. 1-4, which show, by way of example, various types of IOLs that can be formed in accordance with the methods of the present invention. The following description will be limited to IOLs, but it should be understood that all of the methods could also be used to form lens blanks from which ICLs and contact lenses could also be fabricated.

Figure. 1 shows a single-piece IOL 10, which means that the IOL shown in Fig. 1 is cut from a single-piece of plastic material. As shown, the IOL 10 is made up of a clear, central optical portion 12 and colored haptics or support loops 14 that project outwardly from the periphery of the optical portion 12. The loops 14 are used to support the IOL in the eye, either in the anterior or posterior chamber in accordance with existing knowledge. As will be described in greater detail below, the IOL 10 shown in Fig. 1 can be cut from blanks that are formed in accordance with the present invention where an outer peripheral portion from which the loops 14 are formed are colored and an inner portion from which the optical portion 12 is formed is clear. The colored portion can range from opaque to transparent as long as it is formed of a color that is easily observable.

The method could also be used to form blanks from which a single-piece IOL 10 as shown in Fig. 2 is formed where a peripheral edge 16 of the optical portion 12 is also colored and formed of the same material that is used to form the haptics or loops 14. This can be done simply by increasing the width of the colored portion on the blanks that are formed in accordance with the following methods.

Another embodiment of a single-piece IOL 10 is shown in Fig. 3 where the peripheral edge 16 around the optical portion 12 is formed of colored material of one type or shade, while colored material that is used to form the loops 14 is formed of a material or shade different from the material used to form the peripheral edge 16.

In Fig. 4, a multi-piece IOL 18 is shown where an optical portion 20 is formed separately from the support loops 22. The loops 22 can be attached to the optical portion 20 by any suitable means such as through the use of heat, laser or ultrasonic welding techniques. As shown, the optical portion 20 includes a peripheral edge 24 which is formed of a material that is colored relative to the clear central portion 26. The haptics 22 can be colored as shown in Fig. 4 or be formed of a clear material.

The IOLs shown in Figs. 1-4 represent IOLs where polymethylmethacrylate (PMMA) is used to form the single-piece lenses 10 or, for multi-piece lenses 18, to form the optical portion 20. For the multi-piece IOLs 18, haptics are typically formed of polypropylene or extruded PMMA. Other types of materials suitable for forming such lenses could also be used in accordance with the invention.

It should be understood that within the scope of the invention as described below, IOLs and ICLs of different configurations and other variations of colored and transparent portions are contemplated as falling within the scope of the invention.

The IOLs shown in Figs. 1-4 are fabricated from pieces of plastic material formed as a composite of separate materials in accordance with methods described in detail below. The blanks, which are designated by the letter B and shown in various configurations in Fig. 5, can be formed in any suitable shape. As shown in Fig. 5A, the blank B is formed as a square, in Fig. 5B as a circle and in Fig. 5C as an ellipse. The basic shapes described in conjunction with the processes shown in Figs. 6-9 are shown as being circular or square, but other shapes that would allow the lens to more readily be fabricated are considered to be within the scope of the invention. As mentioned above, the lens blanks B can be used to form either single piece or multi-piece lenses as shown.

The embodiments described in detail below use polymerization techniques to form a composite of clear and colored materials. Such methods have advantages over an extrusion method for forming a composite from which a lens blanks can be made. These advantages include the fact that the methods discussed below can be used to form IOLs that are more versatile and may perform better than those formed from known extrusion processes, which are necessarily limited to existing commercially available extrudable polymers.

Further, instead of a mechanical bond that is provided by the extrusion processes between the clear optic portion and the peripheral colored portions, the portions are joined through polymerization, where there is a slight diffusion of adjacent materials. This results in the composite being formed as an integrated rod with no microvoids or weak points at the interface of different materials.

The versatility is illustrated by the fact that polymerization can be tailored to obtained extremely pure and high molecular weight plastics as lens optic, which results in better performance. Plastics with higher molecular weights are more resistant to YAG laser damage than lower molecular weight plastics that are typically used in extrusion processes.

Small amount of active ingredients or functional monomers, e.g., hydroxyl ethyl methacrylate (HEMA), can be mixed in and copolymerized with the main ingredients. This may result in IOLs with improved surface properties and also allow further treatment of IOLs to take place permanently through chemical bonding with the active functional groups. The active ingredient can either be incorporated into optic portion, haptic portion, or both, depending on the need of specific application.

Additionally, the polymerization process allows one to start fabrication of IOLs from low molecular weight monomers or prepolymers which are easier to mix with colorants or dyes than commercially available extrusion material because of lower viscosity. Thus, a wider variety of dyes or colorants can be used in conjunction with lenses formed with the following methods.

Referring to Fig. 6, a casting process for forming an elongated composite C (see Fig. 6C) is schematically illustrated, where an elongated, transparent rod 28 formed of PMMA is provided. The rod 28 has a cross-sectional diameter approximately 5-10 mm, but the size can vary depending on the type of lens that is to be formed from blanks cut from the composite C and whether the optic portions are totally clear or only partially clear with a colored periphery, as described above.

The rod 28 can be formed by mixing 50g of redistilled monomer (MMA) and 0.10g of USP-245 in a cylindrical test tube that has an inner diameter of about 15 mm. USP-245 also known as 2,5-dimethyl-2,5-bis(2-ethyl hexoyl peroxy) hexane is available from Witco Chemical Corporation, U.S. Peroxygen Divison, 850 Morton Avenue, Richmond, California 94804. Methyl Methacrylate (MMA) is avilable from Morton Thiokol, Inc., Alfa Division, 152 Andover Street. Danvers, Massachusetts 01923. The solution is then purged with $N_2$ gas for ten minutes. The test tube is then sealed and placed in an oil bath at a temperature of 60°C for eight hours. The tube is removed from the bath and postcured in oven at 85°C for twelve hours.

After the rod has cooled to room temperature, the test tube is broken away to produce a clear PMMA rod which is milled to a diameter of about 6mm or other preferred diameter. The rod 28 is then positioned or suspended in the center of a mold 30, as shown in Fig. 6B, which has an inner diameter of about 10-25 mm depending on the style of lens to be made. Preferably, the mold 30 has a diameter of about 22 mm to provide a radial distance 32 between the outer surface of the rod 28 and inner surface of the mold 30 of approximately 8 mm.

A colored solution consisting of 50 g of redistilled MMA, 0.10 g of USP-245 and 0.0040 g of Pyla-Cert Black MX453 is poured into the mold 30 as shown schematically by spout 34. In this way, the solution is poured around the rod 28. The materials are allows to set up and polymerize to form an integral, colored, peripheral coating 36 over the rod 28, in order to form the composite C as shown in Fig. 6C. Preferably, the colored solution includes a predistilled monomer (MMA) solution or a prepolymer solution, along with an initiator and dye. A preferred dye is Pyla-Cert Black MX453 is available from PYLAM Products, Inc., 1001 Stewart Avenue, Garden City, New York 11530. A preferred initiator is USP 245, as discussed above.

The outer surface of the composite C is machined (not shown) so that the composite has a predetermined and uniform cross-sectional diameter, which is preferably 20 mm. The composite C is radially sliced into thin discs about 2.2 mm in thickness, as shown by the broken lines in Fig. 6C, to form lens blanks B as shown in Fig. 6D. The blanks are then fabricated into IOLs by known lathe cutting or other techniques (not shown).

As shown in Fig. 6D, the blank B includes an optic portion 38 and a colored peripheral portion 40. If the peripheral portion 40 is large enough, a single-piece lens of the types shown in Figs. 1 or 2 could be formed. If a narrower peripheral edge 40 is provided, an optic suitable for use in the formation of a multi-piece lens as shown in Fig. 4 could be formed. In the formation of a single-piece lens of the type shown in Fig. 2, for example, the optic portion 38 of the lens blank B shown in Fig. 6D would correspond to the optical portion 12 of the IOL 10, and the peripheral rim 16 and supporting loops 14 would be formed from the colored peripheral portion 40 of the lens blank B.

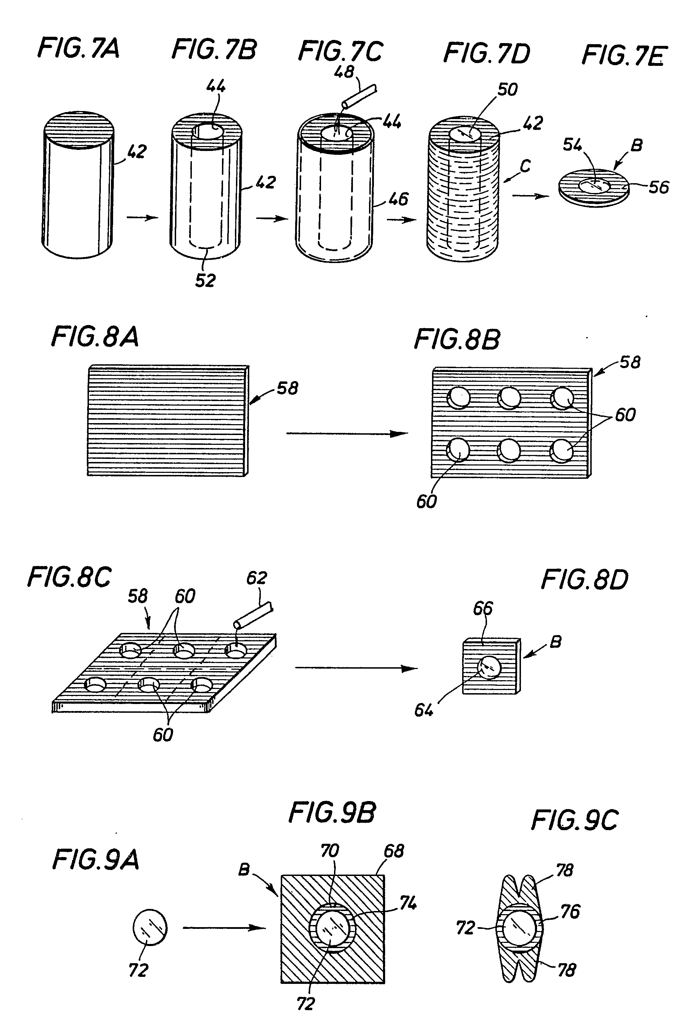

An alternate process of forming IOLs as shown in Fig. 7, where the composite C can be formed by initially casting a colored cylindrical rod 42 (see Fig. 7A), having a diameter of preferably 25 mm in cross-section. The rod 42 can be formed as described in conjunction with the initial casting step of the rod 28 as shown in Fig. 6, with the addition of the suitable dye to the starting solution.

As shown in Fig. 7B, the center of the rod 42 is bored to provide a central bore hole 44 that has a diameter of approximately 5-10 mm. The rod 42 is then placed in a suitable holder 46 (see Fig. 7C) and an MMA monomer solution is poured into the central bore hold 44 by means of, for example, a nozzle 48. The MMA monomer solution is polymerized to the surrounding portion of the rod 42 to form a clear central portion 50 of the composite C as shown in Fig. 7D. The bore hold 44 can include a closed end 52 at the bottom of the rod 42 to hold the monomer solution in place. The resulting composite C can be sliced into a number of lens blanks B as described above, all of which have a clear central portion 54 and surrounding colored portion 56.

Referring to Fig. 8, another method for fabricating IOL blanks in accordance with the present invention is described. This method starts with a sheet of colored PMMA 58 (see Fig. 8A), which can be purchased commercially or prepared by casting as discussed above in connection with the methods of Figs. 6 and 7. A plurality of openings 60 are formed in the sheet 58 as shown in Fig. 8B. The sheet 58 is placed in a mold or other suitable fixture (now shown) and the openings 60 filled with an MMA monomer solution as illustrated by the nozzle 62 shown in Fig. 8C. The MMA monomer or other prepolymer solution is polymerized to the material of the sheet 58, using the same process discussed above.

The sheet 58 is then cut into a suitable number of lens blanks B, as illustrated by the dotted lines in Fig. 8C, all of which include a transparent optic portion 64 and surround colored portion 66.

Another method for fabricating IOL blanks in accordance with the invention is shown in Fig. 9 where a square or disc 68 (see Fig. 9B) formed of colored PMMA is directly cast by pouring a MMA and dye solution into a mold with a desired configuration and then allowed to solidify as discussed above. An opening 70 is formed in the square 68 which is larger in diameter than the diameter of a clear plastic portion 72 as shown in Fig. 9A. The clear plastic portion 72 is placed concentrically within the opening 70 and a colored plastic as described above is poured into the annulus or space 74 that is formed between the clear plastic portions 72 and the edge of the square 68 that forms the opening 70. In this way, a composite is formed with two different types of colored material surrounding a clear central optic portion from which IOLs such as those shown in Fig. 3 can be formed.

A similar composite with more than one colored layer could be formed by using the composites shown in Figs. 6 and 7, by forming another colored layer to the composite using, for example, the method shown in Fig. 6.

As shown in Fig. 9C, a suitable IOL can be fabricated from a double colored lens blank B, where a central optical portion 72 is surrounded by a first peripheral edge 76 that is colored and by a second colored material that is used to form support loops or haptics 78.

Alternatively, a single surrounding colored portion would be formed as discussed above in conjunction with the embodiments shown in Figs. 6-8. In accordance with the invention, the inner colored ring 74 could be of the same color or different colors as the material in the outer portion of the blank B. Also, for example, the inner layer 74 could be formed of a hard plastic material along with the optical portion 72 while the outer colored portion could be a different plastic.

Furthermore, the present invention is not limited to any particular polymerization technique and/or any particular plastics. Free radical polymerization, condensation polymerization, ionic polymerization or a combination of any of the above two techniques may be applied to fabricate one piece IOLs with composite materials. For example, lens blank with polycarbonate optic portion and colored polycarbonate haptic portion may be joined through condensation polymerization and the corresponding IOL fabricated through conventional means. IOLs with PMMA optic and polystyrene haptic may also be joined using free radical polymerization technique and fabricated similarly if such a combination is suitable for any specific application.

Various modifications and improvements to the disclosed invention will become apparent to those with ordinary skill in the art and all such modification or improvements are considered to be included within the scope of the invention are defined by the appended claims.

## Claims

1. A method of forming a composite lens blank (B) from which a lens (10, 18) adapted for use with the human eye can be formed, comprising the steps of:

    (a) providing a first portion (28, 42, 58) formed of a solid, biocompatible plastic that can be sterlized;

    (b) positioning a liquid biocompatible plastic adjacent to the first portion to form a second portion (36, 50, 64), the plastics being selected so that the first and second portions will be bonded through polymerization, said liquid biocompatible plastic can be sterilized in solid form;

    (c) step (b) including positioning one of the portions outwardly of the other portion, at least the material of the outer portion (36, 42, 58) being colored and the material of the inner portion (28, 50, 64) being optically transparent;

    (d) hardening the material of the second portion (36, 50, 64) to form a polymerized composite (c).

2. The method of claim 1, wherein step (a) includes forming an elongated section (28) of clear plastic.

3. The method of claim 2, wherein step (a) includes casting the section (28) in the shape of a rod.

4. The method of claim 2, wherein step (b) includes centering the elongated section (28) in a mold (30) and leaving a space (32) between the elongated section (28) and the mold (30), and pouring colored, liquid plastic into said space (32) to form an elongated composite (c).

5. The method of claim 1, wherein step (a) includes casting an elongated section (42) of colored plastic.

6. The method of claim 5, wherein step (a) includes casting the section (42) in the shape of a rod.

7. The method of claim 5, wherein step (b) includes forming an elongated opening (44) along the length of the rod (42), closing the bottom (52) of the opening and pouring clear plastic material (50) into said opening.

8. The method of claims 4 or 7, and further including slicing the composite (c) into individual lens blanks (B).

9. The method of claim 1, wherein step (a) includes forming a flat sheet (58) of colored plastic.

10. The method of claim 9, wherein step (b) includes forming at least one opening (60) in the sheet (58) and pouring clear liquid into said opening (60) to form a composite.

11. The method of claim 10, wherein the step of forming includes forming a plurality of openings (60) in said sheet (58), the step of pouring including pouring clear liquid into each opening (60), and further including the step of cutting the sheet (58) into a plurality of lens blanks (B).

12. The method of any of the preceding claims and further including the step of bonding through polymerization a third portion formed of a solid, biocompatible plastic that can be sterilized, outwardly of the first and second portions.

FIG.1    FIG.2    FIG.3    FIG.4

FIG.5A    FIG.5B    FIG.5C

FIG.6A    FIG.6B    FIG.6C    FIG.6D

FIG.7A   FIG.7B   FIG.7C   FIG.7D   FIG.7E

FIG.8A   FIG.8B

FIG.8C   FIG.8D

FIG.9B

FIG.9A   FIG.9C